# EUROPÄISCHE PATENTSCHRIFT

(11) **EP 1 411 891 B1**
(45) Veröffentlichungstag und Bekanntmachung des Hinweises auf die Patenterteilung: **09.11.2005**
(21) Anmeldenummer: 02791445.6
(22) Anmeldetag: 05.07.2002
(51) Int. Cl.: A61K 7/48, A61K 7/42

(54) **KOSMETISCHE FORMULIERUNG ENTHALTEND DIHYDROXYACETON**
COSMETIC FORMULATION CONTAINING DIHYDROXYACETONE
FORMULATION COSMETIQUE CONTENANT DE L'ACETONE DIHYDROXY

(30) Priorität: 31.07.2001 DE 10137260
(43) Veröffentlichungstag der Anmeldung: 28.04.2004
(73) Patentinhaber: Merck Patent GmbH, 64293 Darmstadt (DE)
(72) Erfinder: HITZEL, Sabine, 64285 Darmstadt (DE); DRILLER, Hans-Jürgen, 64823 Gross-Umstadt (DE)
(86) Internationale Anmeldenummer: PCT/EP2002/007522
(87) Internationale Veröffentlichungsnummer: WO 2003/011240

(56) Entgegenhaltungen:
- WO-A-94/23693
- DE-A- 19 505 154
- DE-A- 19 834 816
- DE-A- 19 933 466
- FR-A- 2 779 958
- US-A- 5 705 145
- EISVOGEL M: "SUN PROTECTION WITH ANTI-AGEING MERCK PUTS ECTOIN TO WORK" COSSMA: COSMETICS, SPRAY TECHNOLOGY, MARKETING, BRAUN FACHVERLAGE, KARLSRUHE, DE, Bd. 2, Nr. 4, April 2001 (2001-04), Seiten 32-33, XP009012023 ISSN: 1439-7676

## Beschreibung

Die Erfindung betrifft kosmetische Formulierungen auf der Basis von Dihydroxyaceton. Diese Formulierungen können beispielsweise zur Selbstbräunung verwendet werden.

Eine mehr oder minder stark ausgeprägte Bräune der Haut gilt in der modernen Gesellschaft als attraktiv und Ausdruck von Dynamik und Sportlichkeit. Eine derartige Bräune kann beispielsweise durch Sonnenbaden oder durch die Verwendung von Selbstbräunern erzielt werden.

Bei der Einwirkung der Sonne auf die menschliche Haut treten neben der erwünschten Wirkung der Sonne, der Bräunung, auch eine Reihe von unerwünschten Nebenwirkungen auf, wie Sonnenbrand oder vorzeitige Hautalterung und Faltenbildung. Bekanntlich wird der gefährlichste Teil der Sonnenstrahlung von den ultravioletten Strahlen mit einer Wellenlänge von weniger als 400 nm gebildet. Ein Teil dieser UV-Strahlung, die UV-A-Strahlung im Bereich von 320 bis 400 nm, bräunt die Haut, aber sie lässt diese auch altern und begünstigt die Auslösung von erythematösen Reaktionen. Sie kann die erythematöse Reaktion bei bestimmten Menschen auch vergrößern oder sogar phototoxische oder photoallergische und irritative Reaktionen auslösen.

Die durch das Einwirken der Sonne auf die menschliche Haut erzielte Bräune bildet jedoch in gewissem Masse auch einen natürlichen Schutz der Haut vor UV-Strahlen, d.h. diejenigen Menschen, die eine schon durch die Einwirkung der Sonne gebräunte Haut besitzen, sind z.B. vor den schädigenden Einflüssen der UV-A-Strahlung besser geschützt als diejenigen Menschen, deren Haut noch nicht durch die Einwirkung der Sonne gebräunt ist.

Es ist z.B. bekannt, dass Verbindungen, die eine Ketol-Gruppierung 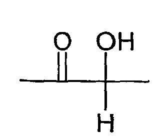 besitzen, wie z.B. Hydroxymethylketone, beipielsweise Methylglyoxal und insbesondere Dihydroxyaceton eine selbstbräunende Wirkung auf die menschliche Haut ausüben und deshalb geeignet sind, in Selbstbräunern verwendet zu werden. Der selbstbräunende Effekt dieser Verbindungen beruht im wesentlichen auf einer Maillard-Reaktion zwischen der Ketol-Gruppierung dieser Verbindungen und den Aminosäuren der Haut.

Es gibt z.B. auch Hinweise darauf, dass die durch Dihydroxyaceton bewirkte Bräunung die Haut ebenfalls vor UV-A-Strahlen schützt [K.A. Follett et al., Dermatologica 175 (1987) 58-63; J.A. Johnson et al., Dermatologica 175 (1987) 53-57; R.A. Wakeel et al., British Journal of Dermatology 1 (1992) 94]. Allerdings ist dieser Schutz vergleichsweise schwach ausgeprägt. Es ist allgemein bekannt, dass der Schutz vor UV-A-Strahlen, der durch die mit Hilfe von Dihydroxyaceton erzielte Bräunung der Haut erzielt wird, nicht so ausgeprägt ist, wie der Schutz, der durch die Sonnenbräune bewirkt wird. Die Anwender von Selbstbräunungszusammensetzungen auf der Basis von Dihydroxyaceton sollten demzufolge verstärkt darauf achten, dass Ihre Haut vor UV- und insbesondere vor UV-A-Strahlen geschützt ist, wenn Sie diesen Strahlen ausgesetzt sind.

Aufgabe der vorliegenden Erfindung war es daher, kosmetische Formulierungen auf der Basis von Dihydroxyaceton zur Verfügung zu stellen, die die Nachteile des Standes der Technik vermeiden und insbesondere aufgetragen auf die Haut zu einem verbesserten UV-A-Schutz führen.

Überraschend wurde nun gefunden, dass diese Aufgabe durch die Verwendung von einer oder mehreren Verbindungen ausgewählt aus den Verbindungen der Formeln la und Ib 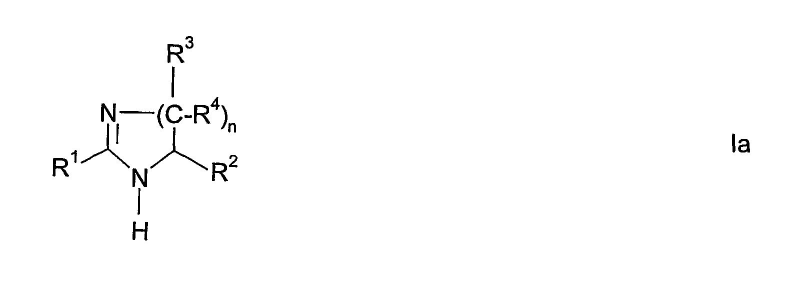 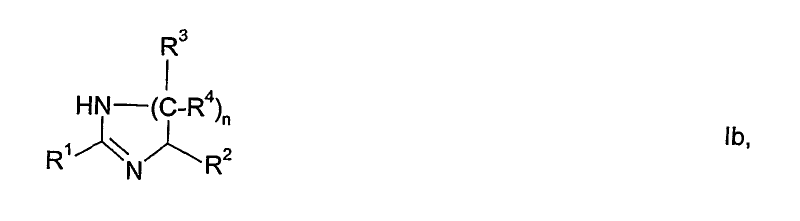 den physiologisch verträglichen Salzen der Verbindungen der Formeln la und Ib, und den stereoisomeren Formen der Verbindungen der Formeln la und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten,
in kosmetischen Formulierungen enthaltend Dihydroxyaceton und einen topischen Träger gelöst wird.

Die Erfindung betrifft somit kosmetische Formulierungen enthaltend Dihydroxyaceton und einen topischen Träger, dadurch gekennzeichnet, dass die Zusammensetzung zusätzlich eine oder mehrere Verbindungen ausgewählt aus den Verbindungen der Formeln la und Ib 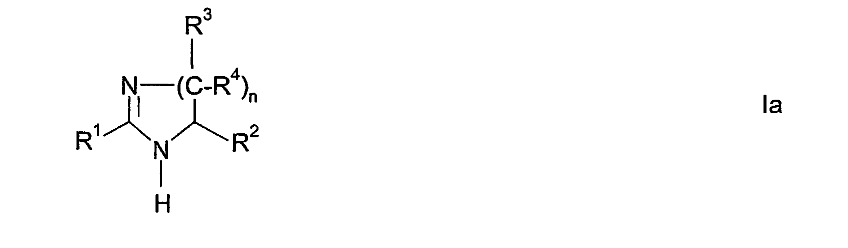 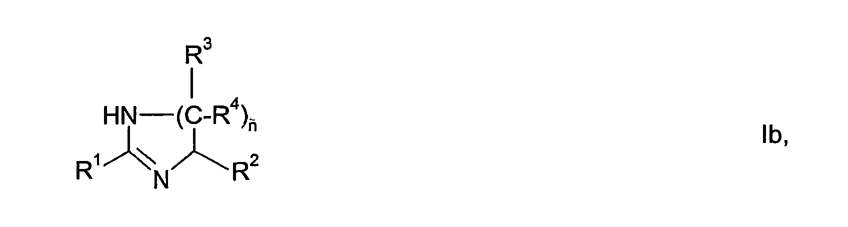 den physiologisch verträglichen Salzen der Verbindungen der Formeln la und Ib, und den stereoisomeren Formen der Verbindungen der Formeln la und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten,
enthält.

Überraschend ist auch, dass die Formulierungen enthaltend Dihydroxyaceton und eine oder mehrere Verbindungen ausgewählt aus den Verbindungen der Formeln la und Ib, den physiologisch verträglichen Salzen der Verbindungen der Formeln la und Ib, und den stereoisomeren Formen der Verbindungen der Formeln la und Ib stabil sind. Es ist nämlich allgemein bekannt, dass Formulierungen enthaltend Dihydroxyaceton und stickstoffhaltige Verbindungen wie z.B. Aminosäuren Stabilitätsprobleme aufweisen. Dies ist beispielsweise auf den Abbau von Dihydroxyaceton in Gegenwart von Aminosäuren zurückzuführen, der bei erhöhter Temperatur sogar noch beschleunigt wird. Es wurde jedoch gefunden, dass Dihydroxyaceton und eine oder mehrere Verbindungen ausgewählt aus den Verbindungen der Formeln la und Ib, den physiologisch verträglichen Salzen der Verbindungen der Formeln la und Ib, und den stereoisomeren Formen der Verbindungen der Formeln la und Ib in kosmetische Formulierungen eingearbeitet werden können, ohne dass Stabilitätsprobleme auftreten oder besondere Vorkehrungen hinsichtlich der Stabilität der resultierenden kosmetischen Formulierung getroffen werden müssten. Dies gilt z.B. innerhalb eines gewissen Rahmens auch für eine erhöhte Lagertemperatur der erfindungsgemässen kosmetischen Formulierungen.

Im Rahmen der vorliegenden Erfindung werden alle vor- und nachstehenden Verbindungen ausgewählt aus den Verbindungen der Formeln la und Ib, den physiologisch verträglichen Salzen der Verbindungen der Formeln la und Ib, und den stereoisomeren Formen der Verbindungen der Formeln la und Ib als "Ectoin oder Ectoin-Derivate" bezeichnet.

Bei Ectoin und den Ectoin-Derivaten handelt es sich um niedermolekulare, cyclische Aminosäurederivate, die aus verschiedenen halophilen Mikroorganismen gewonnen oder synthetisch hergestellt werden können. Sowohl Ectoin als auch Hydroxyectoin besitzen den Vorteil, dass sie nicht mit dem Zellstoffwechsel reagieren.

Die Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib können in den erfindungsgemässen kosmetischen Formulierungen als optische Isomere, Diastereomere, Racemate, Zwitterionen, Kationen oder als Gemisch derselben vorliegen. Unter den Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib, sind diejenigen Verbindungen bevorzugt, worin R¹ H oder CH₃, R² H oder COOH, R³ und R⁴ jeweils unabhängig voneinander H oder OH und n 2 bedeuten. Unter den Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib sind die Verbindungen (S)-1,4,5,6-Tetrahydro-2-methyl-4-pyrimidincarbonsäure (Ectoin) und (S,S)-1,4,5,6-Tetrahydro-5-hydroxy-2-methyl-4-pyrimidincarbonsäure (Hydroxyectoin) insbesondere bevorzugt.

Die im Rest R⁵ der Verbindungen der Formeln la und Ib genannten Aminosäurereste leiten sich von den entsprechenden Aminosäuren ab. Unter dem Begriff "Aminosäure" werden die stereoisomeren Formen, z.B. D- und L-Formen, folgender Verbindungen verstanden: Alanin, β-Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin, γ-Aminobutyrat, Nε-Acetyllysin, Nδ-Acetylornithin, Nγ-Acetyldiaminobutyrat und Nα-Acetyldiaminobutyrat. L-Aminosäuren sind bevorzugt. Die Reste folgender Aminosäuren sind bevorzugt: Alanin, β-Alanin, Asparagin, Asparaginsäure, Glutamin, Glutaminsäure, Glycin, Serin, Threonin, Valin, γ-Aminobutyrat, Nε-Acetyllysin, Nδ-Acetylornithin, Nγ-Acetyldiaminobutyrat und Nα-Acetyldiaminobutyrat.

Die im Rest R⁵ der Verbindungen der Formeln la und Ib genannten Di- und Tripeptidreste sind ihrer chemischen Natur nach Säureamide und zerfallen bei der Hydrolyse in 2 oder 3 Aminosäuren. Die Aminosäuren in den Di- und Tripeptidresten sind durch Amidbindungen miteinander verbunden. Bevorzugte Di- und Tripetidreste sind aus den bevorzugten Aminosäuren aufgebaut.

Die in den Resten R¹, R² und R⁵ der Verbindungen der Formeln la und Ib genannten Alkylgruppen umfassen die Methylgruppe CH₃, die Ethylgruppe C₂H₅, die Propylgruppen CH₂CH₂CH₃ und CH(CH₃)₂ sowie die Butylgruppen CH₂CH₂CH₂CH₃, H₃CCHCH₂CH₃, CH₂CH(CH₃)₂ und C(CH₃)₃. Die bevorzugte Alkylgruppe ist die Methylgruppe.

Bevorzugte physiologisch verträgliche Salze der Verbindungen der Formeln la und Ib sind beispielsweise Alkali-, Erdalkali- oder Ammoniumsalze, wie Na-, K-, Mg- oder Ca-Salze, sowie Salze abgeleitet von den organischen Basen Triethylamin oder Tris-(2-hydroxy-ethyl)-amin. Weitere bevorzugte physiologisch verträgliche Salze der Verbindungen der Formeln la und Ib ergeben sich durch Umsetzung mit anorganischen Säuren wie Salzsäure, Schwefelsäure und Phosphorsäure oder mit organischen Carbon- oder Sulfonsäuren wie Essigsäure, Citronensäure, Benzoesäure, Maleinsäure, Fumarsäure, Weinsäure und p-Toluolsulfonsäure.

Verbindungen der Formeln la und Ib, in denen basische und saure Gruppen wie Carboxyl- oder Aminogruppen in gleicher Zahl vorliegen, bilden innere Salze.

Die Herstellung der Verbindungen der Formel la und Ib ist in der Literatur beschrieben (DE 43 42 560). (S)-1,4,5,6-Tetrahydro-2-methyl-4-pyrimidincarbonsäure oder (S,S)-1,4,5,6-Tetrahydro-5-hydroxy-2-methyl-4-pyrimidincarbonsäure können auch mikrobiologisch gewonnen werden (Severin et al., J. Gen. Microb. 138 (1992) 1629-1638).

Die Verwendung von Verbindungen ausgewählt aus den Verbindungen der Formeln la und Ib für kosmetische und auch pharmazeutische Zwecke ist bereits bekannt.

Beispielsweise wird in der WO 94/15923 beschrieben, dass (S)-1,4,5,6-Tetrahydro-2-methyl-4-pyrimidincarbonsäure oder (S,S)-1,4,5,6-Tetrahydro-5-hydroxy-2-methyl-4-pyrimidincarbonsäure zur Herstellung einer kosmetischen Zubereitung oder eines Arzneimittels, beispielsweise zur Behandlung von Hautkrankheiten, verwendet werden können.

Desweiteren wird in der DE 43 42 560 die Verwendung von Ectoin und Ectoin-Derivaten als Feuchtigkeitsspender in Kosmetikprodukten beschrieben. Diese Produkte eignen sich z.B. zur Pflege von gealteter, trockener oder gereizter Haut.

In der DE 199 33 466 wird desweiteren beschrieben, dass Ectoin und Derivate wie Hydroxyectoin als Antioxidantien und Radikalfänger in kosmetischen und dermatologischen Zubereitungen eingesetzt werden können. Die Zubereitungen können zur Behandlung und/oder Prophylaxe der durch oxidative Beanspruchung hervorgerufenen Hautalterung und von entzündlichen Reaktionen verwendet werden.

Weitere Anwendungen von Ectoin und Ectoinderivaten in kosmetischen Formulierungen werden z.B. in der WO 00/07558, WO 00/07559 und WO 00/07560 beschrieben, wie z.B. die Pflege und Prophylaxe einer trockenen und/oder schuppigen Haut, der Schutz der menschlichen Haut gegen Trockenheit und/oder hohe Salzkonzentrationen, der Schutz von Zellen, Proteinen und/oder Biomembranen der menschlichen Haut, der Schutz der Mikroflora der menschlichen Haut, die Stabilisierung der Hautbarriere sowie der Schutz und die Stabilisierung der Nukleinsäuren von menschlichen Hautzellen.

US 5 705 145 offenbart Dihydroxyaceton und Imidazol enthaltende Zusammensetzungen.

Es war bisher jedoch nicht bekannt, dass die Verbindungen ausgewählt aus den Verbindungen der Formeln la und Ib vorteilhafterweise in kosmetischen Formulierungen enthaltend Dihydroxyaceton verwendet werden können und dass sie z.B. die UV-A-protektive Wirkung von Dihydroxyaceton signifikant verstärken können.

Gegenstand der vorliegenden Erfindung ist daher auch die Verwendung einer oder mehrerer Verbindungen ausgewählt aus den Verbindungen der Formeln la und Ib zur Verstärkung der UV-A-protektiven Wirkung von Dihydroxyaceton, insbesondere in kosmetischen Formulierungen.

Die erfindungsgemässen kosmetischen Formulierungen eignen sich aber beispielsweise auch für die obengenannten und aus dem Stand der Technik bekannten Anwendungen von Ectoin und seinen Derivaten. Die Verwendung der erfindungsgemässen kosmetischen Formulierungen für diese Anwendungen ist deshalb ebenfalls Gegenstand der vorliegenden Anmeldung.

Beispiele für die erfindungsgemässen kosmetischen Formulierungen stellen u.a. Selbstbräunungszusammensetzungen, Produkte für die Tagespflege und Lichtschutzformulierungen dar. In einer bevorzugten Ausführungsform werden die erfindungsgemässen kosmetischen Zusammensetzungen als Selbstbräuner verwendet.

Der Anteil an Dihydroxyaceton in der erfindungsgemässen kosmetischen Formulierung beträgt bevorzugt von 0,1 bis 15Gew.%, besonders bevorzugt von 0,1 bis 10 Gew.% und insbesondere bevorzugt von 1 bis 7 Gew.% bezogen auf die gesamte Formulierung. Ganz ausserordentlich bevorzugt beträgt der Anteil an Dihydroxyaceton in der erfindungsgemässen kosmetischen Formulierung von 2 bis 5 Gew.% bezogen auf die gesamte Formulierung.

Der Anteil der Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib in der erfindungsgemässen kosmetischen Formulierung beträgt vorzugsweise von 0,001 bis 50 Gew.%, besonders bevorzugt von 0,01 bis 10 Gew.% und insbesondere bevorzugt von 0,1 bis 10 Gew.% bezogen auf die gesamte kosmetische Formulierung. Ganz ausserordentlich bevorzugt beträgt der Anteil der genannten Verbindungen in der erfindungsgemässen kosmetischen Formulierung von 0,1 bis 5 Gew.% bezogen auf die gesamte Formulierung.

Die erfindungsgemässen kosmetischen Formulierungen können neben Dihydroxyaceton und den Verbindungen der Formel I auch weitere kosmetische Wirkstoffe enthalten.

Die erfindungsgemässen kosmetischen Formulierungen können ein oder mehrere Antioxidantien enthalten. In den erfindungsgemässen kosmetischen Formulierungen können alle gängigen Antioxidantien enthalten sein. Es gibt in diesem Zusammenhang viele aus der Fachliteratur bekannte und bewährte Substanzen, die verwendet werden können, z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid), Metabisulfitsalze, Sulfitsalze oder Hydrogensulfitsalze.

Mischungen von Antioxidantien sind ebenfalls zur Verwendung in den erfindungsgemässen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Eine weitere geeignete Antioxidantienmischung kann z.B. bestehen aus u.a. Emblicanin-A, Emblicanin-B, Punigluconin und Pendunculagin wie z.B. in der WO 00/48551 unter der Bezeichnung CAPROS™ beschrieben (z.B. Emblica™).

In einer bevorzugten Ausführungsform der Erfindung enthält die erfindungsgemässe kosmetische Formulierung eine oder mehrere Verbindungen ausgewählt aus Flavonoiden und/oder Coumaranonen.

Als Flavonoide werden die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin), α-Glycosylrutin, Tilirosid sowie deren Sulfaten und Phosphaten. Unter den Flavonoiden sind insbesondere Rutin und Troxerutin bevorzugt.

Unter den Coumaranonen ist 4,6,3',4'-Tetrahydroxybenzylcoumaranon-3 bevorzugt.

In einer weiteren bevorzugten Ausführungsform der Erfindung, insbesondere wenn die Wasserlöslichkeit der Flavonoide und/oder Coumaranone gesteigert werden soll, ist an eine oder an mehrere Hydroxygruppen dieser Verbindungen eine polare Gruppe gebunden, z.B. jeweils unabhängig voneinander eine Sulfat- oder Phosphatgruppe. Geeignete Gegenionen sind beispielsweise die Ionen der Alkali- oder Erdalkalimetalle, wobei diese z.B. aus Natrium oder Kalium ausgewählt sind.

Viele Flavonoide und Coumaranone kommen beispielsweise in der Natur vor. Wenn die erfindungsgemässe kosmetische Formulierung derartige Verbindungen enthält, können diese auch durch Extraktion entsprechender Pflanzen gewonnen werden und entweder aufgereinigt als Einzelsubstanz oder auch in Form des gegebenenfalls weiter aufbereiteten Extrakts in die kosmetische Formulierung eingebracht werden.

Der Anteil der einen oder mehreren Verbindungen ausgewählt aus Flavonoiden und Coumaranonen in der erfindungsgemässen kosmetischen Formulierung beträgt vorzugsweise von 0,001 bis 5 Gew.%, besonders bevorzugt von 0,01 bis 2 Gew.% bezogen auf die gesamte Formulierung.

In einer weiteren bevorzugten Ausführungsform der Erfindung enthält die erfindungsgemässe kosmetische Formulierung eine oder mehrere Antioxidantien ausgewählt aus den Substanzen Zitronensäure, Milchsäure, Äpfelsäure, EDTA, Butylhydroxytoluol, Ascorbinsäure, Ascorbylpalmitat, Magnesium-Ascorbylphosphat, Ascorbylacetat, Tocopherol, Tocopherolacetat sowie aus den Metabisulfit-, Sulfit- oder Hydrogensulfitsalzen, die aus Alkalimetallsalzen wie Natrium- und Kaliumsalzen, basischen Metallsalzen und Ammoniumsalzen ausgewählt sind.

In einer weiteren bevorzugten Ausführungsform der Erfindung enthält die erfindungsgemässe kosmetische Formulierung Antioxidantienmischungen wie z.B. Emblica™.

Der Anteil des einen oder der mehreren Antioxidantien in der erfindungsgemässen kosmetische Formulierung beträgt vorzugsweise von 0,001 bis 5 Gew.%, besonders bevorzugt von 0,01 bis 2 Gew.% bezogen auf die gesamte Formulierung.

In den erfindungsgemässen kosmetischen Formulierungen können ein oder mehrere UV-Filter enthalten sein. Als geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, z.B.
Benzylidenkampferderivate wie
   - 3-(4'-Methylbenzyliden)-dl-kampfer (z.B. Eusolex® 6300),
   - 3-Benzylidenkampfer (z.B. Mexoryl® SD),
   - Polymere von N-{(2 und 4)-[(2-oxoborn-3-yliden)methyl]benzyl}-acrylamid (CAS-Nr. 113783-61-2, z.B. Mexoryl® SW),
   - N,N,N-Trimethyl-4-(2-oxoborn-3-ylidenmethyl)anilinium methylsulfat (CAS-Nr. 52793-97-2, z.B. Mexoryl® SK) oder
   - α-(2-Oxoborn-3-yliden)toluol-4-sulfonsäure (CAS-Nr. 56039-58-8, z.B. Mexoryl® SL),
Benzoyl- oder Dibenzoylmethane wie
   - 1-(4-tert-Butylphenyl)-3-(4-methoxyphenyl)propan-1,3-dion (z.B. Eusolex® 9020) oder
   - 4-Isopropyldibenzoylmethan (z.B. Eusolex® 8020),
Benzophenone wie
   - 2-Hydroxy-4-methoxybenzophenon (z.B. Eusolex® 4360) oder
   - 2-Hydroxy-4-methoxybenzophenon-5-sulfonsäure und ihr Natriumsalz (z.B. Uvinul® MS-40),
Methoxyzimtsäureester wie
   - Methoxyzimtsäureoctylester (z.B. Eusolex® 2292),
   - 4-Methoxyzimtsäureisopentylester, z.B. als Gemisch der Isomere (z.B. Neo Heliopan® E 1000),
Salicylatderivate wie
   - 2-Ethylhexylsalicylat (z.B. Eusolex® OS),
   - 4-Isopropylbenzylsalicylat (z.B. Megasol®) oder
   - 3,3,5-Trimethylcyclohexylsalicylat (z.B. Eusolex® HMS),
4-Aminobenzoesäure und Derivate wie
   - 4-Aminobenzoesäure,
   - 4-(Dimethylamino)benzoesäure-2-ethylhexylester (z.B. Eusolex® 6007),
   - ethoxylierter 4-Aminobenzoesäureethylester (z.B. Uvinul® P25),
Benzimidazolderivate wie
   - 2-Phenylbenzimidazol-5-sulfonsäure sowie ihre Kalium-, Natrium- und Triethanolaminsalze (z.B. Eusolex® 232),
   - 2,2'-(1,4-Phenylen)bis-(1H-benzimidazol-4,6-disulfonsäure, Mononatriumsalz) (CAS-Nr. 180 898-37-7, z.B. Neo Heliopan® AP),
   - 2,2'-(1,4-Phenylen)bis-(1H-benzimidazol-5-sulfonsäure) sowie ihre Kalium-, Natrium- und Triethanolaminsalze,
   und weitere Substanzen wie
   - 2-Cyano-3,3-diphenylacrylsäure-2-ethylhexylester (z.B. Eusolex® OCR),
   - 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX),
   - 2,4,6-Trianilino-(p-carbo-2'-ethylhexyl-1'-oxi)-1,3,5-triazin (z.B. Uvinul® T 150),
   - 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
   - 4,4'-[(6-[4-((1,1-Dimethylethyl)aminocarbonyl)phenylamino]-1,3,5-triazin-2,4-diyl)diimino]bis(benzoesäure-2-ethylhexylester) (CAS-Nr. 154702-15-5, z.B. Uvasorb® HEB),
   - α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1, z.B. Parsol® LX),
   - 2,2'-Methylen-bis-(6-(2H-benzotriazol-2-yl)-4-(1,1,3,3-tetramethylbutyl)phenol) (CAS-Nr. 103 597-45-1, z.B. Tinosorb® M),
   - 2,4-bis-{[4-(2-Ethyl-hexyloxy)-2-hydroxyl]-phenyl}-6-(4-methoxyphenyl)-1,3,5-triazin (CAS-Nr. 187 393-00-6, z.B. Tinosorb® S),
   - 2-(4-Diethylamino-2-hydroxy-benzoyl)-benzoesäure hexylester (z.B. Uvinul®UVA Plus).

Die in der Liste aufgeführten Verbindungen sind nur als Beispiele aufzufassen. Selbstverständlich können auch andere UV-Filter verwendet werden. Diese organischen UV-Filter werden wie auch die Verbindungen der Formel I in der Regel in einer Menge von 0,5 bis 20 Gew.-%, vorzugsweise in einer Menge von 1 bis 15 Gew.-% und insbesondere bevorzugt in Mengen von 2 bis 8 Gew.-% je Einzelsubstanz in die erfindungsgemässe kosmetischen Formulierungen eingearbeitet. Insgesamt enthalten die erfindungsgemässen kosmetischen Formulierungen üblicherweise bis zu 40 Gew.-%, vorzugsweise 5 bis 25 Gew.-% solcher organischer UV-Filter.

Als anorganische UV-Filter sind solche aus der Gruppe der Titandioxide wie z.B. gecoatetes Titandioxid (z.B. Eusolex® T-2000, Eusolex® T-AQUA), Zinkoxide (z.B. Sachtotec®), Eisenoxide oder auch Ceroxide denkbar. Diese anorganischen UV-Filter werden in der Regel in einer Menge von 0,5 bis 20 Gew.-%, vorzugsweise 2 bis 10 Gew.-%, in die erfindungsgemässen kosmetischen Formulierungen eingearbeitet.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10 - 10:1, vorzugsweise im Bereich 1:5 - 5:1 und insbesondere bevorzugt im Bereich 1:2 - 2:1. Werden UV-A- neben UV-B-Filtern eingesetzt, so ist es für die meisten Anwendungen von Vorteil, wenn der Anteil an UV-B-Filtern überwiegt und das Verhältnis von UV-A-Filtern : UV-B-Filtern im Bereich 1:1 bis 1:10 liegt.

In den erfindungsgemässen kosmetischen Formulierungen werden als bevorzugte Verbindungen mit UV-filternden Eigenschaften 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und gecoatetes Titandioxid verwendet.

Die erfindungsgemässe kosmetische Formulierung kann auch - abgesehen von den Verbindungen der Formeln la und Ib - eine oder mehrere Aminosäuren oder deren pharmazeutisch verträgliche Salze enthalten.

Bevorzugte Aminosäuren sind ausgewählt aus der Gruppe der Verbindungen bestehend aus Alanin, Valin, Leucin, Isoleucin, Prolin, Methionin, Phenylalanin, Tryptophan, Glycin, Serin, Threonin, Cystein, Tyrosin, Asparagin, Glutamin, Lysin, Arginin und Histidin.

Enthält die erfindungsgemässe Formulierung von den Verbindungen der Formeln la und Ib verschiedene Aminosäuren, so beträgt der Anteil an diesen Aminosäuren oder deren pharmazeutisch verträglichen Salzen in der erfindungsgemässen kosmetischen Formulierung vorzugsweise von 0,1 bis 10 Gew.%, besonders bevorzugt von 0,1 bis 8 Gew.% und insbesondere bevorzugt von 0,2 bis 5 Gew.% bezogen auf die gesamte Formulierung. Ganz ausserordentlich bevorzugt beträgt der Anteil an diesen Aminosäuren oder deren pharmazeutisch verträglichen Salzen in der erfindungsgemässen kosmetischen Formulierung von 0,2 bis 2 Gew.% bezogen auf die gesamte Formulierung.

Wenn die erfindungsgemässe Formulierung von den Verbindungen der Formeln la und Ib verschiedene Aminosäuren enthält, ist der pH-Wert der Formulierung vorzugsweise kleiner 4.

Die Inhaltsstoffe können in der üblichen Weise in die erfindungsgemässen kosmetischen Formulierungen eingearbeitet werden. Geeignet sind Formulierungen für eine äusserliche Anwendung, beispielsweise als Creme, Lotion, Gel, oder als Lösung, die auf die Haut aufgesprüht werden kann. Dabei ist es bevorzugt, wenn die Formulierung mindestens eine Öl- und mindestens eine Wasser-Phase enthält.

Als Anwendungsform der erfindungsgemässen kosmetischen Formulierungen seien z.B. genannt: Lösungen, Emulsionen, PIT-Emulsionen, Suspensionen, Salben, Gele, Cremes, Lotionen, Sprays und Aerosole. Weitere Anwendungsformen sind z.B. Sticks. Der Formulierung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer, Filmbildner, Verdickungsmittel, Feuchthaltemittel.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Die Emulsionen können in verschiedenen Formen vorliegen. So können sie z.B. eine Emulsion oder Mikroemulsion vom Typ Wasser-in-Öl (W/O), oder vom Typ Öl-in-Wasser (O/W), oder eine multiple Emulsion, beispielsweise vom Typ Wasser-in-Öl-in-Wasser (W/O/W), darstellen.

Die erfindungsgemässen kosmetischen Formulierungen können auch als emulgatortreie, disperse Zubereitungen, vorliegen. Sie können beispielsweise Hydrodispersionen oder Pikkering-Emulsionen darstellen.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Pasten, Salben, Gele und Cremes können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Sprays können die üblichen Treibmittel, z.B. Chlorfluorkohlenwasserstoffe, Propan/Butan oder Dimethylether, enthalten.

Weitere typisch kosmetische Anwendungsformen sind z.B. Make-up wie z.B. Emulsions-Make up sowie Sonnenschutz-, Prä-Sun- und After-Sun-Präparate.

Die erfindungsgemässe kosmetische Formulierung eignet sich besonders zum Schutz menschlicher Haut vor den schädigenden Einflüssen der UV-Anteile im Sonnenlicht, daneben bieten sie auch Schutz gegen Alterungsprozesse der Haut sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Formulierung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Creme oder als Milch vorliegt und Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemässe kosmetische Formulierung kann auch als alkoholisches Gel vorliegen, welches einen oder mehrere Niedrigalkohole oder -polyole, wie Ethanol, Propylenglycol oder Glycerin, und ein Verdickungsmittel wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Zum Schutz der Haut vor Sonnenstrahlen wird auf die Haut eine erfindungsgemässe kosmetische Formulierung aufgetragen.

Die erfindungsgemässen kosmetischen Formulierungen können mit Hilfe von Techniken hergestellt werden, die dem Fachmann wohl bekannt sind.

Alle Verbindungen oder Komponenten, die in den erfindungsgemässen kosmetischen Formulierungen verwendet werden können, sind entweder bekannt und käuflich erwerbbar oder können nach Methoden, die dem Fachmann wohl bekannt und in der Literatur beschrieben sind (z.B. in Standard-Werken wie Houben-Weyl, Methoden der organischen Chemie, Georg-Thieme-Verlag, Stuttgart), gewonnen oder hergestellt werden.

Die folgenden Beispiele sollen die vorliegende Erfindung verdeutlichen.

Die INCI-Namen der verwendeten Rohstoffe sind wie folgt (die INCI-Namen werden definitionsgemäss in Englischer Sprache angegeben):

| **Rohstoff** | **INCI-Name** |
|---|---|
| Cetiol® | Oleyl Oleate |
| Dihydroxyaceton | Dihydroxyacetone |
| Dow Corning® 3225 C | Cyclomethicone, Dimethicone Copolyol |
| Emulgator E 2155 | Steareth-10, Steareth-7, Stearyl Alcohol |
| Imwitor® 900 | Glyceryl Stearate |
| Luvitol® EHO | Cetearyl Octanoate |
| Methyl-4-hydroxybenzoat | Methylparaben |
| Miglyol® 812 N | Caprylic/Capric Triglyceride |
| Paracera® M | Microwax |
| Propandiol-1,2 | Propylene Glycol |
| Propyl-4-hydroxybenzoat | Propylparaben |
| RonaCare™ Ectoin | Ectoin |
| Teginacid® H | Glyceryl Stearate, Ceteth-20 |
| Wasser, demineralisiert | Aqua (Water) |

### Beispiele

### Beispiel 1

### Selbstbräunungslotion (O/W)

| | | | **Gew.%** |
|---|---|---|---|
| **A** | Emulgator E 2155 | (1) | 3,0 |
| | Teginacid® H | (1) | 3,0 |
| | Imwitor® 900 | (2) | 3,0 |
| | Paracera® M | (3) | 1,0 |
| | Cetiol® | (4) | 8,5 |
| | Luvitol® EHO | (5) | 11,5 |
| | Miglyol® 812 N | (6) | 8,5 |
| | Propyl-4-hydroxybenzoat | (7) | 0,05 |
| | | | |
| **B** | 1,2-Propandiol | (7) | 4,0 |
| | Wasser, demineralisiert | | 42,2 |
| | Methyl-4-hydroxybenzoat | (7) | 0,15 |
| | | | |
| **C** | Dihydroxyaceton | (7) | 5,0 |
| | RonaCare™ Ectoin | (1) | 0,1 |
| | Wasser, demineralisiert | | 10,0 |

### Herstellung

Die Phase A wird auf 75 °C und die Phase B wird auf 80 °C erhitzt. Anschliessend wird Phase B unter Rühren langsam zu Phase A gegeben und homogenisiert. Bei etwa 40 °C wird Phase C zugegeben und unter Rühren abkühlen gelassen.

### Bezugsquellen

(1) Th. Goldschmidt AG
(2) Hüls AG
(3) Paramelt
(4) Cognis GmbH
(5) BASF AG
(6) Condea Chemie GmbH
(7) Merck KGaA

### Beispiel 2

### Selbstbräunungsmilch (W/O)

| | | | **Gew.%** |
|---|---|---|---|
| **A** | Dow Corning® 3225 C | (2) | 23,6 |
| | | | |
| **B** | Dihydroxyaceton | (1) | 5,0 |
| | Propandiol-1,2 | (1) | 35,9 |
| | Konservierungsmittel | | q.s. |
| | RonaCare™ Ectoin | (1) | 1,0 |
| | Wasser, demineralisiert | | ad 100 |

### Herstellung

Phase B wird gelöst und anschliessend in Phase A eingerührt.

Als Konservierungsmittel werden verwendet:
0,05 % Propyl-4-hydroxybenzoat
0,15 % Methyl-4-hydroxybenzoat

### Bezugsquellen

(1) Merck KGaA
(2) Dow Corning

## Patentansprüche

1. Kosmetische Formulierung enthaltend Dihydroxyaceton und einen topischen Träger, **dadurch gekennzeichnet, dass** die Zusammensetzung zusätzlich eine oder mehrere Verbindungen ausgewählt aus den Verbindungen der Formeln la und Ib 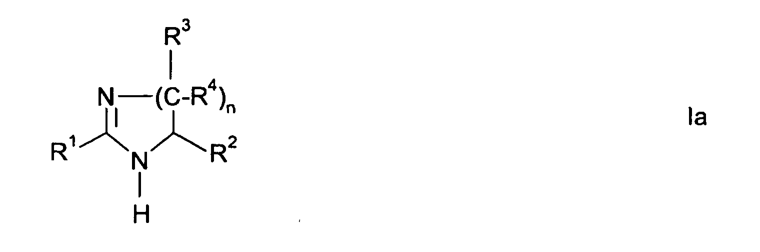 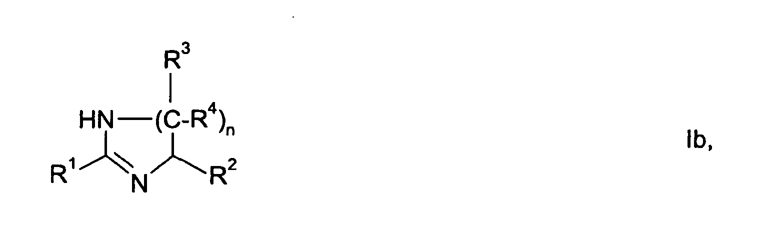 den physiologisch verträglichen Salzen der Verbindungen der Formeln la und Ib, und den stereoisomeren Formen der Verbindungen der Formeln la und Ib, wobei
R¹ H oder Alkyl,
R² H, COOH, COO-Alkyl oder CO-NH-R⁵,
R³ und R⁴ jeweils unabhängig voneinander H oder OH,
n 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten, enthält.

2. Kosmetische Formulierung nach Anspruch 1, **dadurch gekennzeichnet, dass** sie 0,1 bis 15 Gew.% Dihydroxyaceton bezogen auf die gesamte Formulierung enthält.

3. Kosmetische Formulierung nach Anspruch 1 oder 2, **dadurch gekennzeichnet, dass** der Anteil der Verbindungen ausgewählt aus den Verbindungen der Formeln la und Ib, den physiologisch verträglichen Salzen der Verbindungen der Formeln la und Ib, und den stereoisomeren Formen der Verbindungen der Formeln la und Ib von 0,001 bis 50 Gew.% bezogen auf die gesamte Formulierung beträgt.

4. Kosmetische Formulierung nach einem oder mehreren der Ansprüche 1 bis 3, **dadurch gekennzeichnet, dass** die Verbindungen der Formeln la und Ib ausgewählt sind aus den Verbindungen (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure.

5. Kosmetische Formulierung nach einem oder mehreren der Ansprüche 1 bis 4, **dadurch gekennzeichnet, dass** sie ein oder mehrere Antioxidantien enthält.

6. Kosmetische Formulierung nach einem oder mehreren der Ansprüche 1 bis 5, **dadurch gekennzeichnet, dass** sie ein oder mehrere UV-Filter enthält.

7. Kosmetische Formulierung nach einem oder mehreren der Ansprüche 1 bis 6, **dadurch gekennzeichnet, dass** es eine Formulierung zur Selbstbräunung, eine Formulierung für die Tagespflege oder eine Lichtschutzformulierung ist.

8. Verwendung einer oder mehrerer Verbindungen ausgewählt aus den Verbindungen der Formeln la und Ib gemäss Anspruch 1 zur Verstärkung der UV-A-protektiven Wirkung von Dihydroxyaceton, insbesondere in kosmetischen Formulierungen.

## Claims

1. Cosmetic formulation comprising dihydroxyacetone and a topical vehicle, **characterised in that** the composition additionally comprises one or more compounds selected from the compounds of the formulae la and Ib 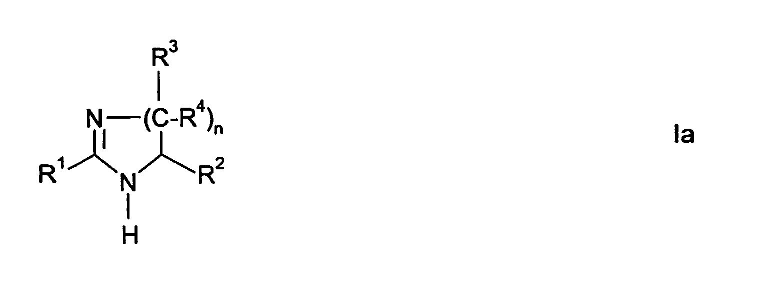 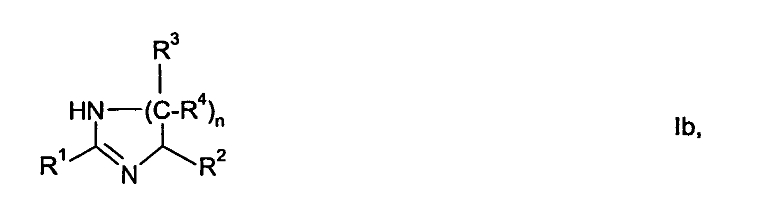 the physiologically tolerated salts of the compounds of the formulae la and Ib, and the stereoisomeric forms of the compounds of the formulae la and Ib, where
R¹ denotes H or alkyl,
R² denotes H, COOH, COO-alkyl or CO-NH-R⁵,
R³ and R⁴ each, independently of one another, denote H or OH,
n denotes 2 or 3,
alkyl denotes an alkyl radical having 1 to 4 carbon atoms, and
R⁵ denotes H, alkyl, an amino acid radical, dipeptide radical or tripeptide radical.

2. Cosmetic formulation according to Claim 1, **characterised in that** it comprises 0.1 to 15% by weight of dihydroxyacetone, based on the formulation as a whole.

3. Cosmetic formulation according to Claim 1 or 2, **characterised in that** the proportion of compounds selected from the compounds of the formulae Ia and Ib, physiologically tolerated salts of the compounds of the formulae Ia and Ib and stereoisomeric forms of the compounds of the formulae Ia and Ib is from 0.001 to 50% by weight, based on the formulation as a whole.

4. Cosmetic formulation according to one or more of Claims 1 to 3, **characterised in that** the compounds of the formulae Ia and Ib are selected from the compounds (S)-1,4,5,6-tetrahydro-2-methyl-4-pyrimidinecarboxylic acid and (S,S)-1,4,5,6-tetrahydro-5-hydroxy-2-methyl-4-pyrimidinecarboxylic acid.

5. Cosmetic formulation according to one or more of Claims 1 to 4, **characterised in that** it comprises one or more antioxidants.

6. Cosmetic formulation according to one or more of Claims 1 to 5, **characterised in that** it comprises one or more UV filters.

7. Cosmetic formulation according to one or more of Claims 1 to 6, **characterised in that** it is a formulation for self-tanning, a formulation for day care or a light-protection formulation.

8. Use of one or more compounds selected from the compounds of the formulae la and Ib according to Claim 1 for augmenting the UV-A-protective action of dihydroxyacetone, in particular in cosmetic formulations.

## Revendications

1. Formulation cosmétique comprenant du dihydroxyacétone et un véhicule topique, **caractérisée en ce que** la composition comprend additionnellement un ou plusieurs composés choisis parmi les composés des formules la et Ib 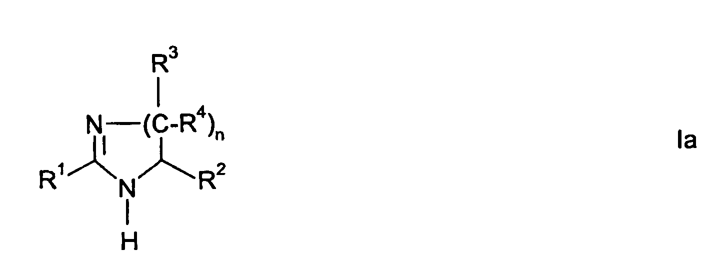 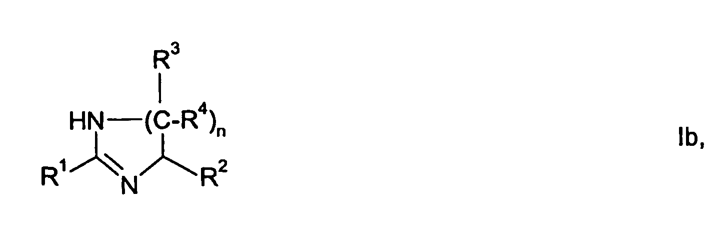 les sels tolérés physiologiquement des composés des formules la et Ib, et les formes stéréo-isomériques des composés des formules la et Ib, où
R¹ représente H ou alkyle,
R² représente H, COOH, COO-alkyle ou CO-NH-R⁵,
R³ et R⁴ représentent, chacun indépendamment l'un de l'autre, H ou OH,
n représente 2 ou 3,
alkyle représente un radical alkyle comportant de 1 à 4 atomes de carbone, et
R⁵ représente H, alkyle, un radical acide aminé, un radical dipeptide ou un radical tripeptide.

2. Formulation cosmétique selon la revendication 1, **caractérisée en ce qu'**elle comprend de 0,1 à 15% en poids de dihydroxyacétone, sur la base de la formulation complète.

3. Formulation cosmétique selon la revendication 1 ou 2, **caractérisée en ce que** la proportion de composés choisis parmi les composés des formules la et Ib, de sels tolérés physiologiquement des composés des formules la et Ib et de formes stéréo-isomériques des composés des formules la et Ib est de 0,001 à 50% en poids, sur la base de la formulation complète.

4. Formulation cosmétique selon une ou plusieurs des revendications 1 à 3, **caractérisée en ce que** les composés des formules la et Ib sont choisis parmi les composés acide (S)-1,4,5,6-tétrahydro-2-méthyl-4-pyrimidinecarboxylique et acide (S,S)-1,4,5,6-tétrahydro-5-hydroxy-2-méthyl-4-pyrimidinecarboxylique.

5. Formulation cosmétique selon une ou plusieurs des revendications 1 à 4, **caractérisée en ce qu'**elle comprend un ou plusieurs antioxydants.

6. Formulation cosmétique selon une ou plusieurs des revendications 1 à 5, **caractérisée en ce qu'**elle comprend un ou plusieurs filtres UV.

7. Formulation cosmétique selon une ou plusieurs des revendications 1 à 6, **caractérisée en ce qu'**il s'agit d'une formulation d'auto-bronzage, d'une formulation de soin de jour ou d'une formulation de protection vis-à-vis de la lumière.

8. Utilisation d'un ou de plusieurs composés choisis parmi les composés des formules la et Ib selon la revendication 1 pour augmenter l'action de protection du dihydroxyacétone vis-à-vis des UV-A, en particulier dans des formulations cosmétiques.
